# EUROPÄISCHE PATENTSCHRIFT

(11) **EP 2 569 141 B1**
(45) Veröffentlichungstag und Bekanntmachung des Hinweises auf die Patenterteilung: **25.10.2017**
(21) Anmeldenummer: 11751795.3
(22) Anmeldetag: 10.05.2011
(51) Int. Cl.: B29C 67/00, A61L 27/38, C12N 5/00

(54) **VERFAHREN ZUR HERSTELLUNG EINER ORGANNACHBILDUNG, INSBESONDERE EINES FUNKTIONSMODELLS**
METHOD FOR PRODUCING AN ORGAN REPLICA, IN PARTICULAR A FUNCTIONAL MODEL
PROCÉDÉ POUR RÉALISER UNE REPRODUCTION D'ORGANE, EN PARTICULIER UN MODÈLE FONCTIONNEL

(30) Priorität: 11.05.2010 DE 102010020222
(43) Veröffentlichungstag der Anmeldung: 20.03.2013
(73) Patentinhaber: Haverich, Axel, 30177 Hannover (DE)
(72) Erfinder: Haverich, Axel, 30177 Hannover (DE)
(74) Vertreter: Gramm, Lins & Partner Patent- und Rechtsanwälte PartGmbB
(86) Internationale Anmeldenummer: PCT/DE2011/001051
(87) Internationale Veröffentlichungsnummer: WO 2011/153981

(56) Entgegenhaltungen:
- WO-A2-2007/106497
- US-A1- 2008 133 040
- A Ovsianikov, M Gruene, M Pflaum, L Koch, F Maiorana, M Wilhelmi, A Haverich, B Chichkov: "Laser printing of cells into 3D scaffolds", IOPSCIENCE , 10. März 2010 (2010-03-10), XP002665511, DOI: 10.1088/1758-5082/2/1/014104 Gefunden im Internet: URL:http://iopscience.iop.org/1758-5090/2/ 1/014104 [gefunden am 2011-12-08]
- Scott J. Hollister: "Scaffold Design and Manufacturing: From Concept to Clinic", Advanced Materials , 2. Juni 2009 (2009-06-02), XP002665512, DOI: 10.1002/adma.200802977 Gefunden im Internet: URL:http://onlinelibrary.wiley.com/doi/10. 1002/adma.200802977/abstract [gefunden am 2011-12-08]
- MIRONOV V ET AL: "Organ printing: Tissue spheroids as building blocks", BIOMATERIALS, ELSEVIER SCIENCE PUBLISHERS BV., BARKING, GB, Bd. 30, Nr. 12, 1. April 2009 (2009-04-01) , Seiten 2164-2174, XP025990541, ISSN: 0142-9612, DOI: 10.1016/J.BIOMATERIALS.2008.12.084 [gefunden am 2009-01-26]
- HARRIS ET AL: "Recent progress in CAD/CAM laser direct-writing of biomaterials", MATERIALS SCIENCE AND ENGINEERING C, ELSEVIER SCIENCE S.A, CH, Bd. 28, Nr. 3, 28. Februar 2008 (2008-02-28), Seiten 359-365, XP022503032, ISSN: 0928-4931, DOI: 10.1016/J.MSEC.2007.04.013

## Beschreibung

Die Erfindung betrifft ein Verfahren zur Herstellung einer Nachbildung eines menschlichen oder tierischen Organs oder eines Teils davon oder eines sonstigen Segments eines menschlichen oder tierischen Körpers, insbesondere als Funktionsmodell für Forschung und Lehre. Seit geraumer Zeit wird versucht, biologische Gewebe naturähnlich nachzubilden, um diese Gebilde einerseits als Modelle für die Forschung und andererseits für medizinische Zwecke bei Patienten mit Gewebeverlust einsetzen zu können. Für das zugehörige Arbeitsgebiet hat sich international die Bezeichnung "tissue engineering" herausgebildet.

Aus der EP 1362092 B1 beispielsweise ist ein Zellpatch auf Basis einer Collagenvliesunterlage bekannt. Die Unterlage aus einem Wundversorgungsvlies mit Collagenfibrillen ermöglicht eine Kultur darauf aufgebrachter Zellen, die teilweise deren Selbstorganisation zulässt. Das damit erhaltene artifizielle Gewebepatch kann für das Studium von Medikamentenwirkungen, die Wirkung von Wachstumsfaktoren auf die Zellkultivierung oder chirurgisch als Augmentationsmaterial verwendet werden. Komplexere Gewebestrukturen können auf diese Weise noch nicht erhalten werden.

Die EP 0989867 B1 offenbart ein grundlegendes Verfahren zur Herstellung einer azellularisierten und rebesiedelten nativen Collagenmatrix als Gewebetransplantat. Das Patent sieht jedoch noch keine aktive Vaskularisierung vor, so dass die Versorgung der Zellen des frisch hergestellten bioartifiziellen Materials ein Problem darstellt.

Dies überwindet die Lehre der EP 1230939 B1, bei der eine primär vaskularisierte, azellularisierte, autologe, allogene oder xenogene Gewebematrix mit wenigstens einem Gefäßast für die Rebesiedlung *in vitro* oder *in vivo* eingesetzt wird. Das neue, bioartifizielle Gewebe ist jedoch an die vaskuläre Struktur der zu Grunde gelegten Matrix gebunden und benötigt eine menschliche bzw. tierische Ausgangsmatrix.

Der bisher zumeist eingeschlagene Weg, eine native extrazelluläre Matrix azellularisiert vorzugeben, künstlich, z.B. mit Polymer nachzubilden oder aus nativem Material zu formen und diese Matrix dann im Körper umbilden zu lassen oder mit einem externen Verfahren vorzubesiedeln, beschränkt die Variabilität der erhältlichen Gewebe in hohem Maße, da komplexe Gewebe technisch nicht handhabbar sind.

Die Modellierung oder Nachbildung bzw. Neukonstruktion ganzer Organe steckt daher in den Kinderschuhen und beschränkt sich auf solche Organe mit zumindest bereichsweise relativ gleichförmiger, nicht zu komplexer Gewebestruktur. Konkrete Ergebnisse konnten bislang in Richtung Haut, Darm und Blase erzielt werden, wie beispielsweise die DE 600 28 616 T2 zeigt.

Die WO 2007/106497 A2 offenbart die 3-dimensionale Herstellung biokompatibler Strukturen für Tissue Engineering und Organersatz. Dabei wird eine Grundlage für das Einwachsen von Zellen erzeugt. Diese Grundlage oder Basisstruktur ist porös und umfasst wenigstens eine Wand, die eine Kavität definiert. Die Basisstruktur kann durch Multi-Photonen-Lithographie hergestellt sein.

Die Veröffentlichung A. Ovsianikov, M. Gruene, M. Pflaum, L. Koch, F. Maiorama, M. Wilhelmi, A. Haverich, B. Chichkov: "Laser printing of cells into 3D scaffolds", in Biofabrication 2 (2010) 014104, S. 1 bis 7 beschreibt die Herstellung hochporöser 3D Strukturen mittels 2-Photonenpolymerisation, auf die Zellen mit Hilfe von LIFT aufgebracht werden. Durch eine vertikale, tubuläre Porenstruktur können die Zellen mittels LIFT besonders tief in die Struktur eingebracht werden. Dabei wird zuerst das Gerüst vollständig aufgebaut und in dieses vollständig vorliegende Gerüst werden dann die Zellen eingebracht, z.B. mit dem LIFT-Verfahren. Es wird beschreiben, dass in tieferen Schichten das Einbringen der Zellen ungenauer bzw. undefinierter wird.

Aus Scott J. Hollister: "Scaffold Design and Manufacturing "From Concept to Clinic", Advanced Materials, 2009, 21, 3330-3342 ist es bekannt, neben CAD auch bildgebende Verfahren für das makroskopische Design der aufzubauenden Struktur zu nutzen. Die Mikrostruktur umfasst die Verteilung von Material und Poren. Beides wird mit Topologie-Optimierungsverfahren (Optistrukt™, Altair Engineering) bezüglich physikalischer Eigenschaften optimiert.

Der Erfindung liegt demgegenüber die Aufgabe zugrunde, unter Nutzung biologischer Materialien, unter anderem von Zellen, naturähnliche Nachbildungen ganzer Organe zur Verfügung stellen zu können. Die Nachbildungen sollen natürliche Funktionen zeigen und somit als Modelle in Forschung und Lehre dienen können (Funktionsmodelle). Die natürlichen Funktionen sollen zumindest in künstlicher Umgebung (in vitro) unter schonenden Bedingungen beobachtbar sein. Ferner sollen die Nachbildungen die Transplantationsforschung befruchten.

Die Aufgabe wird durch ein Verfahren nach Anspruch 1 gelöst. Weitere vorteilhafte Ausgestaltungen der Erfindung sind in den Unteransprüchen gekennzeichnet.

Zur Lösung der Aufgabe geht die Erfindung einen ganz neuen Weg. Die Erfindung nutzt die Entwicklungen in der Rapid Prototyping-Technik und insbesondere in der Stereolithographie und daraus abgeleiteten Verfahren zur dreidimensionalen Konstruktion immer feinerer, auch biologischer Strukturen, um ausgewählte Materialien für die Konstruktion eines Organ-Funktionsmodells im Raum gezielt so zu positionieren, dass sich eine Nachbildung eines Organs oder eines Segments, beispielsweise in Form eines Modells, ergibt. Die für die Nachbildung ausgewählten Materialien, u. a. Zellen, Matrixmaterialien, wie Kollagen, Fibrin, Polysaccharide usw., werden dabei anhand von zuvor gewonnener Daten des nachzubildenden Organs oder Segments, die in Datensätzen vorliegen und eine Art von Kartierung bilden, an ganz bestimmte Positionen navigiert.
Die Erfindung nutzt die Fähigkeit von Zellen und biologischen Materialien zur Selbstorganisation. Hierauf wird unter anderem auch bei bereits bekannten Beschichtungsverfahren vertraut, wenn lebende Zellen in einer Suspension auf eine Unterlage gegeben und dort kultiviert werden. Auch dort bilden sich Zellverbände selbstständig aus, was beispielsweise durch die Zugabe von Faktoren (Wachstumsfaktoren u.a.) unterstützt werden kann.

"Auffüllverfahren" im Sinne der Erfindung bedeutet, dass mit dem Verfahren (beim Prototyping) erzeugte Hohlräume mit weiteren Materialien, Flüssigkeiten, stützenden Feststoffen usw. aufgefüllt werden. Hierbei können auch Zellen positioniert werden, z.B. wenn dabei Zellen in die Hohlräume eingebracht werden, die sich in einem Nährmedium befinden, welches die Zellen nicht sedimentieren lässt (z.B. ein halbfestes Medium - Agar oder Gelatine enthaltend).

In den nachzubildenden Organen vorhandene Freiräume - Lumina, Kavitäten - lassen sich für ein Stützgerüst nutzen, indem dort zunächst ein Stützmaterial platziert wird, das später aus der - zum Beispiel durch Selbstorganisation der biologischen Materialien - in sich stabiler gewordenen Nachbildung bzw. dem Modell des Organs entfernt werden kann. Bei den Stützmaterialien kann es sich beispielsweise um auf dem Arbeitsgebiet bekannte, vorzugsweise leicht wieder zu solubilisierende Gele oder beispielsweise Zuckerpolymere handeln.

Das allgemeine erfindungsgemäße Verfahren wird nachfolgend in größerem Detail erläutert.

Grundsätzlich ist das erfindungsgemäße Verfahren zur Herstellung einer Nachbildung eines menschlichen oder tierischen Organs oder eines Teils davon oder eines sonstigen Körpersegments durch die Schritte a) bis d) gekennzeichnet, was nicht ausschließt, dass weitere Schritte hinzukommen können, beispielsweise Schritte, die der Stabilisierung der neu gewonnenen Gewebe dienen, oder Spülschritte.

Unter dem Begriff "Organteile" werden z.B. einzelne Lungenlappen, einzelne Leberlappen, einzelne Hautareale, Blutgefäße etc. verstanden. Im Einzelnen umfasst das Verfahren immer (explizit oder implizit) die Schritte:
a) Bereitstellen vorab erfasster strukturgeometrischer Daten des nachzubildenden Organs, Organteils oder Segments;
b) Auswahl von Rekonstruktionsmaterialien für das Organ oder den nachzubildenden Teil, die wenigstens folgende Gruppen umfassen: wenigstens ein Material zur Rekonstruktion einer extrazellulären Matrix, wenigstens eine Art organspezifischer Zellen,
c) Zuordnung der strukturgeometrischen Daten zu den einzelnen gewählten Rekonstruktionsmaterialien;
d) schichtweiser Aufbau des Modells durch aufeinander folgende Anwendung ortsselektiver Aufbauverfahren für wenigstens einen Teil der Rekonstruktionsmaterialien anhand eines zugehörigen Datensatzes, wobei ausschließlich ortsselektive Aufbauverfahren angewendet werden oder diese mit weiteren Verfahren wie Beschichtungsverfahren oder Auffüllverfahren kombiniert werden.

In Schritt a) werden vorab erfasste strukturgeometrische Daten des nachzubildenden Organs, Organteils oder Segments bereitgestellt.

Unter "strukturgeometrischen Daten" wird die detaillierte Geometrie des nachzubildenden Originalkörperteils, also Organs oder Segments, in Form von 3D-Daten verstanden. Die Strukturgeometrie ist dabei die Geometrie, d.h. die Raumform der biologischen Struktur, auch bezüglich ihrer Details im Inneren.

Grundsätzlich muss zunächst zu jedem Punkt im Raum die dort vorliegende biologische Beschaffenheit des als Vorlage dienenden Organs, Organteils oder Segments festgestellt werden. Aufgrund der tatsächlichen Auflösung der hierzu verwendeten Verfahren handelt es sich de facto nicht um Raumpunkte, sondern um kleinste kubische Raumzonen, auch wenn im Folgenden von Punkten gesprochen wird. Für einfachere Modelle ist es auch möglich, die Auflösung bewusst zu vergröbern, um so die Nachbildung zu vereinfachen.

Die "strukturgeometrischen Daten" bilden demnach einen Datensatz aus einer Angabe zu einem Materialtyp oder einer Materialklasse in Zuordnung zu dessen dreidimensionaler Positionierung im Raum, und zwar konkret innerhalb der Einhüllenden des nachzubildenden Organs, Organteils oder Segments. Die Daten können in einem Gesamtdatensatz oder in mehreren Einzeldatensätzen erfasst werden oder vorliegen, wie nachstehend noch erläutert. Die Minimalanforderung für die Daten der Datensätze ist daher eine Form (Mᵢ; Xᵢ; yᵢ; zᵢ) mit i = 1 bis n; n €{N}, wobei M ein Wert für die Beschaffenheit des Materials ist und x, y, und z die Raumkoordinaten darstellen.

Die Gewinnung der strukturgeometrischen Daten stellt insofern einen dem eigentlichen Konstruktions- oder Nachbildungsverfahren vorausgehenden Verfahrensschritt dar, da diese Daten nicht für jede Konstruktion einer Nachbildung neu erhoben werden müssen. Vielmehr ist derzeit vorgesehen, dass Standard-Organformen, zum Beispiel von Herz, Lunge, Niere, Leber, Bauchspeicheldrüse, Muskeln, Zähnen, nachgebildet werden, so dass im Einzelfall die einmalige Datenerhebung genügen kann. Selbstverständlich ist es möglich mehrere Standardtypen zu erfassen, so z.B. das gleiche Organ in verschiedenen Stadien der Entwicklung oder des Auswachsens oder in geschlechtsspezifischer Ausbildung.

Als Vorlage, d.h. für die Erfassung der strukturgeometrischen Daten, werden natürliche Organe benötigt. Bevorzugt wird das Verfahren auf Herzen, Herzteile und Gefäße angewendet.

Grundsätzlich werden die strukturgeometrischen Daten nicht-invasiv am lebenden Körper ermittelt, beispielsweise spektroskopisch, unter anderem über MRT (Magnetresonanztomografie), über Echografie oder Computertomografie. Eine weitere Möglichkeit besteht darin, ein natürliches Organ zu präparieren und die strukturgeometrischen Daten aus dem meist isolierten, präparierten Organ zu gewinnen. Im Falle von Tierorganen erhält man diese beispielsweise vom Schlachthof. Für menschliche Organe, Organteile oder sonstige Segmente, die klassisch nicht als Organe bezeichnet werden, die jedoch mit dem erfindungsgemäßen Verfahren nachgebildet werden können, kommen Körperspenden oder gesunde Organe aus der klinischen Pathologie in Frage. Für die Nachbildung eines Herzens zu einer Nachbildung, d.h. einem Modell oder Implantat nach der Erfindung, benötigt man beispielsweise wenigstens ein Frauenherz, ein Männerherz und ein Kinderherz. Neben menschlichen Organen können auch Tierorgane verwendet und verschiedenste Tierorganmodelle nachgebildet werden, beispielsweise können Herzen von Schweinen, Gorillas oder Schimpansen verwendet werden.

Eine alternative Möglichkeit zur Ermittlung der strukturgeometrischen Daten besteht daher erfindungsgemäß darin, das nachzubildende, als Vorlage dienende Organ zu präparieren, mit einem Schnittmedium, beispielsweise einem Wachs oder Harz, zu tränken und aus dem Organ Schnitte, vorzugsweise Ultramikrotomschnitte, herzustellen, wobei zur Bestimmung der unterschiedlichen biologischen Materialien vor oder nach dem Schneiden angefärbt wird. Vorzugsweise wird eine Immunhistologische Färbung verwendet.

Die strukturgeometrischen Daten werden dann erfindungsgemäß aus den angefärbten Schnittbildern gewonnen, indem die unterschiedlichen Materialien dieser Schnittbilder identifiziert und ihre Koordinaten festgehalten werden. Bezogen auf den Einzelschnitt werden unmittelbar Flächenkoordinaten erhalten, bezogen auf die Schnittfolge Raumkoordinaten.

Für die Anfärbung kommen verschiedenste Standardverfahren in Betracht, die auch kombiniert werden können, um ein vollständiges Bild zu erhalten. Die Färbeverfahren sind dem Fachmann auf dem Gebiet, einem Biologen, Biochemiker oder Mediziner, bekannt und brauchen daher hier nicht näher ausgeführt zu werden. Geeignet scheinen unter anderem histologische Verfahren, wie die Hämatoxylin-Eosin-Färbung (HE), eine Übersichtsfärbung zur Unterscheidung verschiedener Gewebestrukturen. Auf diese Weise erhält man bereits eine Zuordnung der Raumkoordinaten für extrazelluläre Matrix (ECM) und Zellen, wobei letztere auf Basis ihres Erscheinungsbildes klassifiziert werden können. Geeignet scheint auch eine PAS-Färbung (Periodic-Acid-Schiff'sreaction) zur Darstellung polysaccharidhaltiger Zell- und Gewebebestandteile. Weiter geeignet scheint z.B. die Ladewig-Färbung (Bindegewebsfärbung, Unterscheidung von Kollagenfasern (blau), Zellkernen (braun), Erys (orange) und Muskulatur (blau)), die Azan-Färbung, die Goldner-Färbung, die Elastica-van Gieson-Färbung, die Gomori- oder die Gitter-Färbung, um nur einige zu nennen.

Über immunhistochemische (immunhistologische) Techniken können Gewebe und Zellen genauer differenziert werden. Dies geschieht über spezifische Antikörper, die selbst mit Fluoreszenzfarbstoffen, Enzymen, partikulärem Material (z. B. Goldpartikeln) oder mit radioaktiven Isotopen markiert sind. Alternativ können spezifische Primärantikörper eingesetzt werden, die über markierte Sekundärantikörper nachgewiesen werden können. Eine erfolgte Markierung wird je nach ihrer Art unterschiedlich nachgewiesen. Im Einzelnen wählt der Fachmann aufgrund seines Fachwissens geeignet Methoden aus.

Die Festlegung der Materialgrenzen kann nun beispielsweise visuell und durch manuelle Eingabe der visuell ermittelten Grenzen innerhalb der Einzelschnittbilder erfolgen. Man erhält einen Gesamtdatensatz mit den Raumkoordinaten einzelner, ausgewählter, bzw. betrachteter Materialien (Bindegewebe, verschiedene Zellen, Flüssigkeitsbereiche, ggf. Leerräume, etc.) Die Zuordnung kann sowohl manuell, d.h. per visueller Inspektion und manueller Dateneingabe, erfolgen, als auch (teil-)automatisiert, ggf. mit visueller/manueller Nachbearbeitung und Überprüfung.

Gemäß einer besonders bevorzugten Ausführungsform der Erfindung kann eine automatische Zuordnung auf Basis von Messwerten aus den Schnittdarstellungen erfolgen. Insbesondere sind Farbwerte, Schwärzungsgrade (dunkel/hell-Erkennung), Messintensitäten oder Kontrastwerte der Einzelpunkte der gefärbten Schnitte für die Zuordnung geeignet.

Für die Aufstellung des Gesamtdatensatzes oder der Datensätze für die Geometriedaten einzelner ermittelter Materialien kann der Fachmann auf Basis seines Fachwissens weitere Verfahren und Kriterien heranziehen.

Direkt bei der Erfassung oder anschließend in einem gesonderten Schritt können mehrere in dem natürlichen Organ, d.h. dem Vorlagepräparat, das nachgebildet werden soll, vorhandene Materialien, die nicht jedes für sich genommen in dem Modell nachgebildet werden können oder sollen, zu einer Materialklasse zusammengefasst werden. So kann es einzelne, in einem Zellverband vorhandene besondere Zellen geben, die so nicht für die Nachbildung, d.h. die Modellkonstruktion, bereitgestellt werden können. Das gesamte Gebiet kann dann vereinfachend mit gleichartigen Zellen dargestellt bzw. nachgebildet werden, die aufgrund ihrer Zugehörigkeit zu einem bestimmten Typ von Zellen, z.B. zum Typ Epithelzelle, Bindegewebszelle, Nervenzelle oder Muskelzelle, ausgewählt werden.

In der oben dargestellten Weise können zunächst die strukturgeometrischen Daten für die natürliche Vorlage als ein vollständiger Datensatz zu allen (erkennbaren) Ausgangsmaterialien gewonnen werden, und diese können dann durch Korrelation zu einem Datensatz für die strukturgeometrischen Daten der Nachbildung umgesetzt werden, d. h. für die genaue dreidimensionale Positionierung jedes einzelnen Rekonstruktionsmaterials, oder die Rekonstruktionsmaterialien können direkt bei der Ermittlung der Strukturdaten erkannten Originalmaterialien zugeordnet werden. In beiden Fällen erhält man einen Gesamtdatensatz für die Rekonstruktionsmaterialien in genauer, dreidimensionaler Zuordnung, der für den nachfolgenden Materialaufbau der Nachbildung verwendet wird.

In Weiterbildung der Erfindung kann es vorgesehen sein, dass die Daten des Gesamtdatensatzes auf Basis ihrer Farbwerte, Kontrastwerte oder sonstigen optischen Daten den Unterdatensätzen für die Rekonstruktionsmaterialien zugeordnet werden.

Unter "Rekonstruktionsmaterialien" werden hier die Materialien verstanden, die in der Nachbildung anstelle der durch die Vorlagenanalyse erkannten Originalmaterialien vorhanden sind. Teils sind dies exakt die gleichen oder entsprechenden Materialien, wie in der Vorlage, teils gezielt anders ausgewählte Materialien. So können z.B. Positionen ("Raumpunkte", s.o.), die im Bild des Vorlageorgans Endothelzellen zeigen, auch im Modell mit Endothelzellen besetzt werden. Andererseits kann auch vorgesehen sein, dass als Rekonstruktionsmaterial für Collagen ein bestimmtes anderes Polymer verwendet wird. Alle Raumpunkte, für die der erhobene Datensatz Collagen anzeigt, würden dann im Modell bzw. in der Nachbildung des Organs oder Segments mit dem ausgesuchten Rekonstruktions-Polymer besetzt.

Nach dem reinen Erfassen der Ursprungsmaterialien in Schritt a) ist es daher in einem weiteren Schritt b) in jedem Fall erforderlich konkrete Rekonstruktionsmaterialien für das Organ oder den nachzubildenden Teil des Organs auszuwählen. Erfindungsgemäß sollen diese wenigstens die folgenden Gruppen umfassen:
1. wenigstens ein Material zur Rekonstruktion einer extrazellulären Matrix. Hierbei muss es sich um ein strukturgebendes Material handeln, beispielsweise Collagen, eine collagenartige oder collagenbildende Masse, die sich verfestigt oder nachträglich verfestigt werden kann, eine faserbildende Masse, eine Hyaluronsäure- oder Hyaluronsäurederivat-Mischung, ein sonstiges Biopolymer, ein synthetisches Polymer, insbesondere ein solches, welches in einem Körper umbildbar, d.h. resorbierbar und natürlich substituierbar ist.
2. wenigstens eine Art organspezifischer Zellen, und zwar vorzugsweise mehrere Arten organspezifischer Zellen, sofern mehrere Arten in dem Organ oder Organteil vorhanden sind. Unter organspezifischen Zellen werden zunächst alle Zellen verstanden, die sich in dem Organ oder Körpersegment finden (einschließlich nicht auf das spezielle Organ beschränkter Zellen). Im Einzelnen sind dies sowohl die parenchymatösen Zellen (z.B. die Hepatozyten der Leber) als auch die Zellen, die das Stroma (Interstitium) eines Organs bilden (z.B. Bindegewebszellen). Eine erste Art von Zellen kann dabei vorzugsweise vom Typ Muskelzellen sein, bevorzugt sind glatte Muskelzellen (SMC), quergestreifte Muskelzellen und Herzmuskelzellen, eine zweite Art von Zellen kann bevorzugt vom Typ Epithelzelle sein, bevorzugt sind Hautepithelzellen, Darmepithelzellen, Pneumozyten (Alveolarepithelzellen), Endothelzellen. Weiter können Zellen vom Typ Nervenzelle (motorische Nervenzellen, sensorische Nervenzellen und Interneurone sowie die Gliazellen (Astrozyten, Oligodendrozyten, Schwann'sche Zellen und Mantel-oder Hüllzellen (Amphizyten)), sowie vom Typ Bindegewebs-/Stützgewebszelle (Fibroblasten, Myofibroblasten,Tendinozyten, Chondrozyten, Osteoblasten, Adipozyten, Perizyten usw.) eingesetzt werden.

Auch können Stammzellen vorgesehen werden, die später *in situ* mit Hilfe bestimmter weiterer Stoffe - u.a. Differenzierungsfaktoren, z.B. bestimmte Hyaluronsäuren - ausdifferenzieren.

In Schritt c) des erfindungsgemäßen Verfahrens werden die strukturgeometrischen Daten den einzelnen für den Modellaufbau gewählten Rekonstruktionsmaterialien zugeordnet. Dies kann explizit nach der Erfassung der wahren Materialien in einem zusätzlichen Schritt erfolgen, oder den Schnittbildern können auf Basis bestimmter Zuordnungskriterien direkt Rekonstruktionsmaterialien zugeordnet werden. In jedem Falle werden die Rekonstruktionsmaterialien auf Basis von Messwerten aus Schnittdarstellungen erkannt und zugeordnet, gleich wie diese erhalten wurden, insbesondere auf Basis von Farbwerten, Schwärzungsgraden, Messintensitäten oder Kontrastwerten.

Wie oben schon erwähnt, können sich die Rekonstruktionsmaterialien von den für den jeweiligen Ort ermittelten biologischen Originalmaterialien unterscheiden. Dies trägt unter anderem der Komplexität des Originals Rechnung, dessen exakte Nachbildung nicht möglich erscheint. Dennoch finden sich in den meisten Organen größere Raumbereiche einer einheitlichen Substanz- oder Zellklasse, die mit Hilfe eines einheitlichen Rekonstruktionsmaterials nachgebildet werden können. Für die Konstruktion der Nachbildung können auch Zellen mit Materialien gemischt werden, die die Bildung eines Zellverbandes fördern sollen - anders als in der Vorlage. Für die Nachbildung bzw. Modellkonstruktion können beispielsweise bestimmte Zellgemische, Mischungen von Zellen und Medien, Mischungen von Zellen und Faktoren usw. verwendet werden. Gleiches gilt auch für die Bereitstellung der Extrazellulären Matrix. Hier können ebenfalls andere Materialien verwendet werden, als im Einzelnen aus der Vorlage (dem Schnitt) zu erkennen. Diese gemischten Rekonstruktionsmaterialien werden dann auf Basis der strukturgeometrischen Daten, d.h. auf Basis der dafür ermittelten Daten oder Datensätze, dem im Modell vorgesehenen Einsatzgebiet zugeordnet. Wesentlich für die Erfindung ist die Zuordnung der Materialien, d.h. eine konkrete räumliche Zuordnung zwischen dem ermittelten Ausgangsmaterial und einem hierfür an gleicher räumlicher Stelle in der Nachbildung, d.h. dem Modell, dem Implantat, der Prothese, vorgesehenen Rekonstruktionsmaterial.

Für die Korrelation zwischen den strukturgeometrischen Ursprungsdaten und den Daten für die Rekonstruktionsmaterialien kann der Fachmann bekannte mathematische Methoden heranziehen und für die Korrelation weitere Kriterien aufstellen.

Beispielsweise ist es möglich, zusätzlich manuell festgelegte Raumkoordinaten - dies können beispielsweise Koordinaten für besonders wichtige Schlüsselpositionen innerhalb der Nachbildung sein - als Eckdaten für eine Korrelation zu verwenden.

Innerhalb der Schnittebenen können Grenzen zwischen den Rekonstruktionsmaterialien durch Interpolation oder Funktionsbeschreibung zwischen vorgegebenen Eckdaten festgelegt werden.

Nach Abschluss der Vorarbeiten gemäß Schritten a) bis c) erfolgt der praktische Aufbau der Nachbildung gemäß Schritt d) mit Hilfe von als solches bekannten ortsselektiven Aufbauverfahren. In der Regel werden mehrere dieser Aufbauverfahren für die Konstruktion einer Nachbildung erforderlich sein. Gemeinsam bilden die Aufbauverfahren, die ausgewählt wurden, ein Prototyping-Verfahren für die Nachbildung bzw. das Modell.

Aufgrund der durch das Erfassen, wichten und werten der Strukturgeometrischen Daten gewonnenen "Karte" des nachzubildenden Organs oder Organteils werden nun, im nachfolgenden Schritt, die ausgewählten Rekonstruktionsmaterialien an ihre zugeordnete Position navigiert.

In Schritt d) des erfindungsgemäßen Verfahrens erfolgt der schichtweise Aufbau durch aufeinander folgende Anwendung ortsselektiver Aufbauverfahren für wenigstens einen Teil der Rekonstruktionsmaterialien anhand jeweils zugeordneter Datensätze. Dies umfasst wenigstens, wie in Schritt b) schon genannt, wenigstens ein Matrixmaterial und die wenigstens eine Art organspezifischer Zellen. Weitere Materialien können mit anderen, zusätzlichen Verfahren - beispielsweise durch Beschichten - in das Rohmodell eingebracht werden. Die für die ortsspezifischen Aufbauverfahren (Prototyping-Verfahren) erforderlichen Datensätze können als Matrizen vorliegen oder in einer anderen mathematischen Aufbereitung, die für die Steuerung des gewünschten Aufbauverfahrens geeignet ist. Generell können die erfassten strukturgeometrischen Daten als Teildatensätze, z.B. für bestimmte räumliche Bereiche, die mit einem bestimmten Aufbauverfahren zu bearbeiten sind, oder für bestimmte Rekonstruktionsmaterialien, vorliegen oder in Form einer Gesamtdatenmatrix, auf die zur Steuerung der Aufbauverfahren gezielt zugegriffen wird. Es können erfindungsgemäß ausschließlich ortsselektive Aufbauverfahren angewendet werden oder diese können mit weiteren Verfahren wie Beschichtungsverfahren oder Auffüllverfahren kombiniert werden. Für bestimmte im Schnitt erkannte Materialien können Leerräume in dem Modell verbleiben, z.B. für Flüssigkeiten wie Blut (innerhalb von Gefäßen) oder falls auch im Originalorgan ein Hohlraum besteht, wie beispielsweise in der Lunge. Es können auch Leerräume beim primären schichtweisen Aufbau dort belassen werden, wo anschließend weitere Aufbauverfahren, wie z.B. die Beschichtung des soweit erhaltenen Modells, eingesetzt werden. Weiterhin können anstelle der endgültigen Rekonstruktionsmaterialien für das Modell zunächst provisorische Ersatzmaterialien verwendet werden, die anschließend durch andere Rekonstruktionsmaterialien ersetzt werden.

In einer bevorzugten Ausführungsform werden aus dem Gesamtdatensatz zunächst Rekonstruktionsmaterial-spezifische Datensätze oder Datenmatrizen für die Konstruktion der Nachbildung erstellt. Dies ist jedoch in erster Linie eine rein technische Maßnahme, um für die verschiedenen Aufbauverfahren, die nachfolgend noch beschrieben werden, gesonderte Datensätze für die Verfahrenssteuerung zu gewinnen. Die Vorrichtungen für die Aufbauverfahren können auch jeweils auf den Gesamtdatensatz zugreifen, soweit dies technisch möglich und angebracht ist.

Die genauere Zuordnung zwischen Ausgangs- und Rekonstruktionsmaterialien und die Korrelation der Daten kann der Fachmann aufgrund seiner Fachkenntnisse im Einzelnen auf verschiedene Weise ausführen.

Wenigstens eines dieser Aufbauverfahren kann ein Stereolithographieverfahren oder ein daraus weiterentwickeltes Verfahren sein, wozu hier insbesondere die Zwei- bzw. Mehrphotonenpolymerisation gezählt wird.

Klassische Stereolithographieverfahren sind beispielsweise aus der WO95/25003 oder der WO 93/08506 bekannt. Es handelt sich dabei um Verfahren zur Herstellung von Formkörpern aus einem durch Bestrahlung zu verfestigenden flüssigen Material in einem Bad dieses Materials, und zwar primär schichtweise aufbauend, durch Bestrahlen der Badoberfläche. Mit Hilfe eines an verschiedenen Raumpunkten des Bades fokussierbaren Laserstrahls konnte diese Form der Stereolithographie weiterentwickelt werden, so dass nun die gezielte Polymerisation innerhalb des Bades allein durch Verschieben des Laserfokusses möglich ist. Zur Beschreibung dieses Verfahrens wird auf die DE 101 11 422 A1 Bezug genommen.

Mit dem Verfahren der Zwei-Photonen-Polymerisation ist es auch möglich, sehr feine Strukturen zu realisieren, wie sie in biologischen Systemen, z.B. in Organen, vorkommen. Es wird Bezug genommen auf die Veröffentlichung "Twophoton polymerization technique for microfabrication of CAD-designed 3D scaffolds from commercially available photosensitive materials", Journal of Tissue Engineering and Regenerative Medicine, 2007, 1, 443-449, A. Ovisianikov, S. Schlie, A. Ngezahayo, A. Haverich und B. N. Chichkov, die hiermit in den Offenbarungsgehalt dieser Anmeldung durch Bezugnahme einbezogen wird.

Insbesondere für die schichtweise Nachbildung (den Aufbau) der extrazellulären Matrix wird vorzugsweise ein Stereolithographieverfahren und besonders vorzugsweise die Zwei- bzw. Mehrphotonenpolymerisation angewendet.

Als ortsselektives Aufbauverfahren für die schichtweise Nachbildung, d.h. den Aufbau, der Zellschichten und -bereiche wird vorzugsweise ein Laser Induced Forward Transfer (LIFT) der Zellen verwendet.

Dabei ist es möglich, die gewünschte Zellart bzw. die als Rekonstruktionsmaterial kultivierten Zellen gleichmäßig, flächig auf den LIFT-Träger aufzubringen und einzelne Zellen von dort punktuell in die aktuell aufzubauende Nachbildungsebene zu transferieren. Die Daten-Matrix, die die zu transferienden Positionen innerhalb der auf den Träger zu beschichtenden Zellen angibt, ist eine Teilmatrix der Rekonstruktionsdaten-Gesamtmatrix, die wiederum aus der Rohdaten-Gesamtmatrix des Ausgangsmodells gewonnen wurde.

Die auf dem LIFT-Träger vorgelegten Zellen können, falls gewünscht, mit einer Vorzugsorientierung versehen sein. Sie können unter Erhalt einer Orientierung transferiert werden.

In alternativer Ausführungsform kann auch das extrazelluläre Matrixmaterial mittels LIFT an seinen Zielort in der Nachbildung gebracht werden. Besonders bevorzugt ist es, wenn jeweils eine komplette Schicht/Schnittebene der Nachbildung mit LIFT aus den verschiedenen Rekonstruktionsmaterialien zusammengesetzt wird.

Mit dem Laser Induced Transfer Verfahren können kleinste Partikel, Moleküle und insbesondere auch Zellen in einer Translationsbewegung gezielt mit Hilfe eines Lasers und dessen Energie von einem Ausgangspunkt zu einem Zielpunkt bewegt werden. Die zu bewegenden Zellen werden dabei beispielsweise in dünner Schicht in Suspension auf einen Glasträger beschichtet, der von der Rückseite mit dem (fokussierten) Laser bestrahlt wird. Unterstützt wird der Transport durch Hilfspartikel, z.B. bestimmte Nanopartikel oder elementares Edelmetall, z.B. Gold, in dünner Schicht, das durch die Laserenergie verdampft und dabei die Zellen oder sonstigen zu transportierenden Materialien von dem Träger fortkatapultiert.

Eine genauere Beschreibung des LIFT-Verfahrens findet sich unter anderem in der US 2009/0130427 A1 oder der WO 03/037606, die zur Beschreibung dieses erfindungsgemäßen Aufbauverfahrens in die Offenbarung dieser Anmeldung einbezogen werden.

Überraschenderweise kann mit dem LIFT-Verfahren ein schichtweiser exakter Aufbau der Zellbereiche der Nachbildung bzw. des Modells erfolgen.

Der Aufbau kann in ebenen Schichten oder alternativ in sphärischen Schichten erfolgen, wobei der Aufbau mit dem LIFT-Verfahren bevorzugt in ebenen Schichten erfolgt.

Um zunächst eine solide Basis zu erhalten, wird in einer bevorzugten Ausführungsform der schichtweise Aufbau an einer ausgewählten Schnittfläche mit nicht zu kleinem Querschnitt begonnen und ab dieser Querschnittsfläche alternierend oder nacheinander (sequentiell) in beide Querrichtungen zur Schnittfläche ausgeführt.

Gemäß einem besonders bevorzugten Ausführungsbeispiel der Erfindung werden die Lumina zur Stabilisierung zunächst mit einem löslichen oder unter milden Bedingungen wieder entfernbaren formstabilen Material, beispielsweise einem Polymer gefüllt. Dies geschieht vorzugsweise, indem während des ortsselektiven Aufbaus an der Position der Lumina - d.h. an Positionen, an denen das nachzubildende Organ gas- oder flüssigkeitsgefüllt ist, insbesondere innerhalb von Blutgefäßen - als Rekonstruktionsmaterial ein festes, mechanische Stabilität verleihendes Polymer eingebracht wird. Aus einem Gefäßast wird so ein zusätzliches Stützgerüst für das Modell (die Nachbildung). Nach Fertigstellen des Modells bzw. der Nachbildung und nachdem die biologischen Materialien ausreichende Eigenstabilität erreicht haben, wird das stabilisierende Material wieder entfernt. Bei dem Stabilisierungsmaterial kann es sich um auf dem Arbeitsgebiet bekannte Gele oder wasserlösliche Polymere, wie beispielsweise bestimmte Polysaccharide oder Zuckerpolymere handeln. Im direkten räumlichen Anschluss an die Lumina können solche Bereiche miterfasst werden, die später durch Beschichten innerhalb der Lumina aufgebracht werden können. Innerhalb eines Gefäßastes ist es beispielsweise gut möglich, Gefäßinnenbeschichtungen nachträglich aufzubringen, z.B. eine Schicht aus Endothelzellen. Diese Innenbeschichtung von Gefäßen geschieht mit als solches bekannten Verfahren, z.B. aus Zellsuspension.

Als strukturgebende Materialien für die Nachbildung und insbesondere als Rekonstruktionsmaterial zur Rekonstruktion der extrazellulären Matrix wird bevorzugt ein Vorläufer für ein durch Energieeinwirkung und besonders vorzugsweise durch Strahlung verfestigbares Polymer eingesetzt. Dies kann ein synthetisches oder ein auf natürlichen Ausgangsstoffen basierendes Polymer sein. Je nach Verwendung der Nachbildung werden langzeitstabile, mechanisch belastbare nicht-resorbierbare Polymere oder alternativ biokompatible, bioresorbierbare oder auch resorbierbare und in einem Körper umbildbare (remodellierbare) Polymere verwendet. Die Polymere können als solche, d.h. in homogener Phase, oder im Gemisch mit ein oder mehreren Biopolymeren bzw. vor der Verfestigung mit Biopolymer-mono- oder Oligomeren verwendet werden. Bevorzugte Rekonstruktionsmaterialien enthalten beispielsweise Hyaluronsäure und/oder Collagen; in Mischungen für Rekonstruktionsmaterialien einsetzbar sind auch ECM-Mischungen, wie beispielsweise Matrigel®. Fibrin kann als Matrixmaterial aus vorzugsweise photopolymerisierbarem Fibrinogen erhalten werden, wie grundsätzlich im Stand der Technik bekannt. Fibrin kann auch klassisch (enzymatisch) in situ polymerisierend bzw. gelierend mit dem LIFT-Verfahren portionsweise übertragen werden.

In bevorzugten Ausführungsbeispielen werden Zellen der folgenden Gruppen als Rekonstruktionsmaterialien verwendet:
vom Typ Muskelzelle (glatte Muskelzellen, quergestreifte Muskelzellen, Herzmuskelzellen),
vom Typ Epithelzelle (Darmepithelzellen, Hautepithelzellen,Darmepithelzellen, Endothelzellen, Pneumozyten, Endothelzellen)
vom Typ Nervenzelle (motorische Neurone, sensorische Neurone, Interneurone, sowie Gliazellen (Astrocyten, Oligodendrozyten, Schwann'sche Zellen, Mantel- oder Hüllzellen (Amphizyten)),
vom Typ Bindegewebszelle (Fibroblasten, Tendinozyten, Chondrozyten, Osteoblasten, Adipozyten, Myofibroblasten, Perizyten)
sowie Stammzellen. Die Aufzählung ist beispielhaft und nicht abgeschlossen. Weitere spezifische Funktionszellen eines Organs, die das Parenchym ausmachen, können ebenfalls in das Modell eingebracht werden, so etwa Hepatozyten für Lebergewebe; B-Zellen (Insulinzellen), A-Zellen (Glucagonzellen), D-Zellen (Somatostatinzellen) für Pankreasgewebe etc.

Als weitere Rekonstruktionsmaterialien können gegebenenfalls, d.h. optional Ionen, Aminosäuren, Peptide, Proteine, Lipide, Kohlenhydrate, Nukleinsäuren, Wachstumsfaktoren, Differenzierungsfaktoren, Nährstoffe, Hormone und/oder Chemotaktika zugefügt werden.

In Weiterbildung der Erfindung ist auch vorgesehen, dass die vorgenannten Zusatzstoffe und gegebenenfalls weitere oder andere Substanzen während der Aufbauschritte zusammen mit den Zellen eingebracht oder nach den Aufbauschritten oder nach einzelnen Aufbauschritten durch Beschichtungsverfahren und/oder Spülen in die (Organ-)Nachbildung eingebracht werden.

Das erfindungsgemäße Verfahren ergibt eine Nachbildung eines menschlichen oder tierischen Organs, eines Teils davon oder eines sonstigen Segments des menschlichen oder tierischen Körpers mit einem naturähnlichen Aufbau mit wenigstens einem Polymer in Ersatz für extrazelluläre Matrix des Organs mit Zellen und/oder Zellverbänden aus wenigstens einer Zellart und mit organspezifischen gas-, flüssigkeits- oder blutgefüllten Freiräumen. Der Aufbau ist im Einzelnen selbstverständlich organspezifisch.

Bevorzugt enthält die Nachbildung wenigstens eine erste Art von Zellen vom Typ Muskelzellen und wenigstens eine zweite Art von Zellen vom Typ Epithelzellen.

Weiter vorzugsweise sind Zellen vom Typ Bindegewebszelle und vom Typ Nervenzelle vorhanden. Die eingebrachten Zellen bilden später ihre eigene Matrix und bilden Interzellularsubstanz, so dass keinerlei unerwünschte Hohlräume verbleiben.

Das Polymer, das das Rekonstruktionsmaterial für die extrazelluläre Matrix bildet oder in diesem Rekonstruktionsmaterial vorhanden ist, ist vorzugsweise ein biologisch abbaubares oder in einem menschlichen oder tierischen Körper umbaubares (remodellierbares) Polymer, wie oben beschrieben.

Die durch das Verfahren erhaltene Nachbildung kann zunächst als Anschauungsmodell und als Funktionsmodell für die Forschung dienen, beispielsweise für die Untersuchung von Medikamentenwirkungen oder den Einfluss aller übrigen Manipulationen an Organen, gleich ob chirurgischer, pharmakologischer oder gentechnischer Art. Weiterhin ist die Nachbildung zur Verwendung als Prothese oder Implantat in einem menschlichen oder tierischen Körper vorgesehen.

Im Folgenden wird die Erfindung anhand von Ausführungsbeispielen und Abbildungen näher erläutert. Diese Erläuterungen sollen allein der Illustration der Erfindung dienen und die Erfindung in ihrer Gesamtheit nicht beschränken.

Hierzu wird auf die Abbildungen Bezug genommen. Es zeigen:
- Abb. 1: einen histologisch angefärbten Ultramikrotomschnitt eines Bereichs eines Schweineherzens;
- Abb. 2:: eine schematische Darstellung der Strukturerkennung an einem stark vergrößerten Schnittbereich;
- Abb. 2a:: Diagramm mit Ortskoordinaten für verschiedene erkannte Zelltypen für einen Ausschnitt aus der Strukturerkennung nach Abb. 2 (siehe Zuordnung)
- Abb. 3:: das prinzipielle Verfahren der 3D-Rekonstruktion aus Einzelschichten bzw. Einzelschnittbildern;
- Abb. 4:: ein im Rechner erzeugtes virtuelles Organschnittbild.

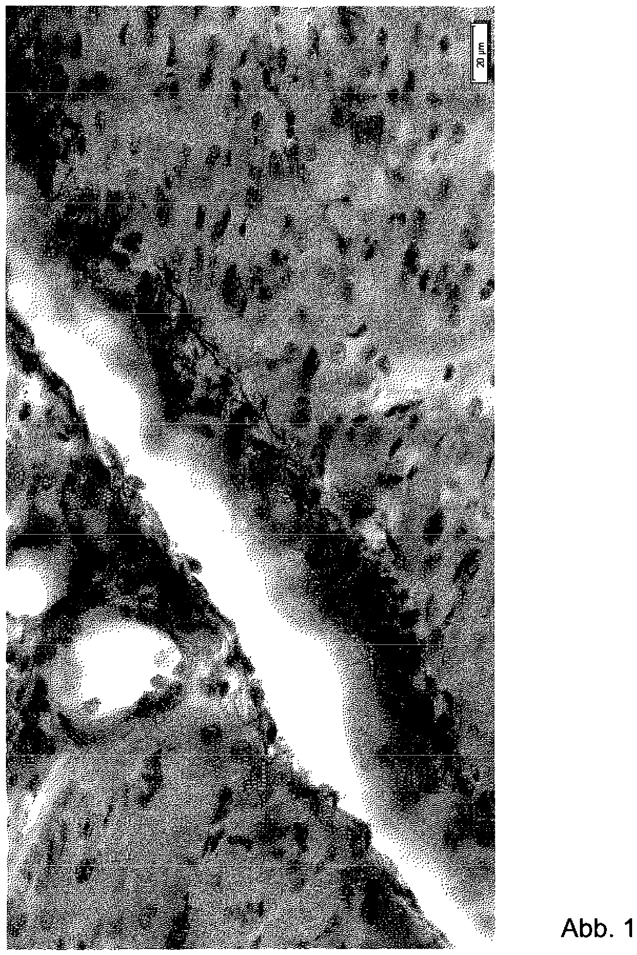

Aus Abbildung 1 ist zu erkennen, wie die mit einer HE-Färbung angefärbten Schnitte eines Organpräparates die genaue räumliche Zuordnung der im Vorlageorgan vorhandenen biologischen Materialien (Zellen, extrazelluläre Matrix, Gefäßlumen) ermöglichen. Aus der flächigen Darstellung erhält man im Rechner durch die Schnittfolge und die Schichtdicke der Schnitte eine dreidimensionale Zuordnung aller ausgewählten, d.h. betrachteten Ursprungsmaterialien. Der Ultramikrotomschnitt kann auch "mehrfarbig" mit mehreren nacheinander angewendeten Methoden angefärbt werden. Dies kann die Zuordnung zu den Rekonstruktionsmaterialien erleichtern. Es ermöglicht auch die automatische Erkennung bestimmter im Vorlage-Organ vorhandener Zellen und Materialien anhand von Farbwerten des Schnittbildes.

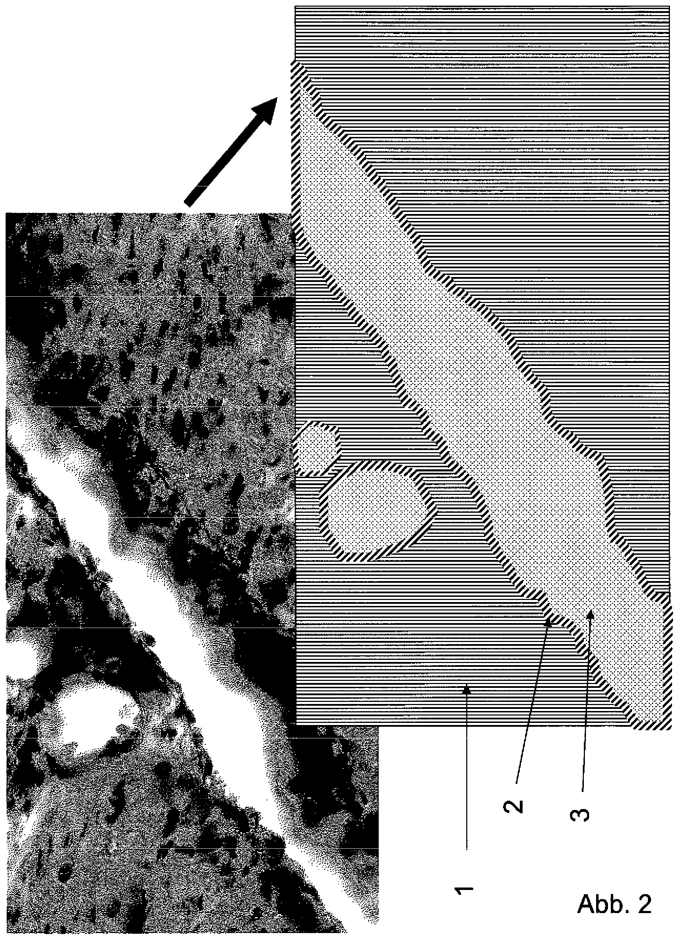

Abbildung 2 zeigt eine vereinfachte Darstellung der Strukturerkennung. Aus dem Ultramikrotomschnitt eines Schweineherzens wurde hier ein stark vergrößerter Ausschnitt gewählt. Aus dem Schnittbild wurde in diesem Beispielfall die genaue - bezüglich der Schnittebene zweidimensionale, flächenmäßige - Position von Muskelzellen 1, Gefäßwände 2 und Gefäßlumina 3 ermittelt und in einem Flächenbild gespeichert. Durch Übereinanderschichten derartiger Flächenbilder entstehen im Computer die dreidimensionalen strukturgeometrischen Daten, die für die Nachbildung, d.h. den Aufbau des Modells benötigt werden.

Abbildung 2a zeigt ein Diagramm mit den Ortskoordinaten bestimmter nach Typ erfasster Zellen in einem Ausschnittsbereich aus der Strukturerkennung nach Abb. 2. Der Ausschnitt, dem das Diagramm zugeordnet ist, ist durch den Rahmen im einkopierten Schnittbild 10 aus Abbildung 2 kenntlich gemacht. Ausgehend von einem Onset oder Ursprung sind die kartesischen Koordinaten innerhalb der Schnittfläche in Mikrometern (µm) angegeben. Die vier erkannten und zugeordneten Zelltypen sind schematisch mit vier verschiedenen Symbolen bezeichnet. Innerhalb eines Rasters, von dem zuvor festgelegt wird, wie grob oder fein es in einem Nano- bis Mikrometerbereich eingestellt wird, kann jedem Rasterpunkt bzw. jeder Rasterfläche innerhalb der Schnittebene ein Material, d. h. hier ein Zelltyp, zugeordnet werden. Entsprechend werden die Rasterpunkte bzw. Rasterflächen am Modell bestimmt - und damit für die Rekonstruktion festgelegt - an denen Vakuolen, Lumina oder Flüssigkeitsbereiche vorhanden sein sollen. Diese Bereiche können übergangsweise während der Rekonstruktion durch Ersatzmaterialien, wie z. B. Stützmaterialien, dargestellt werden. Stützmaterialien können beispielsweise Gelatine oder andere, wieder entfernbare Polymere sein. Es sind jedoch auch Rekonstruktionen möglich, bei denen Lumina nicht aufgefüllt werden müssen, wenn diese beispielsweise so von in sich formstabilen Materialien, z. B: bestimmten Matrixmaterialien, wie Kollagen, umgeben sind, dass der Hohlraum fixiert ist, das Organ-Rekonstrukt also an dieser Stelle während der schichtweisen Rekonstruktion nicht zusammenfällt.

Die Zellzuordnungssymbole in Abbildung 2a haben folgende Bedeutung:

**Tabelle 1**

| Symbol | "erkannter" Zelltyp/-Materialtyp | für die Rekonstruktion ausgewählter Materialtyp |
|---|---|---|
| X | Endothelzellen | Endothelzellen (standardisiert oder (empfänger)spezifisch kultiviert) |
| ■ | SMCs längs zur Bildebene | SMCs - nicht orientiert |
| + | SMCs senkrecht zur Bildebene | SMCs - nicht orientiert |
| Δ | andere Zellen, vorwiegend Fibroblasten | Fibroblasten |
| Leer (luminal) | (Lumen) | ggf. Stabilisierungsmaterial |

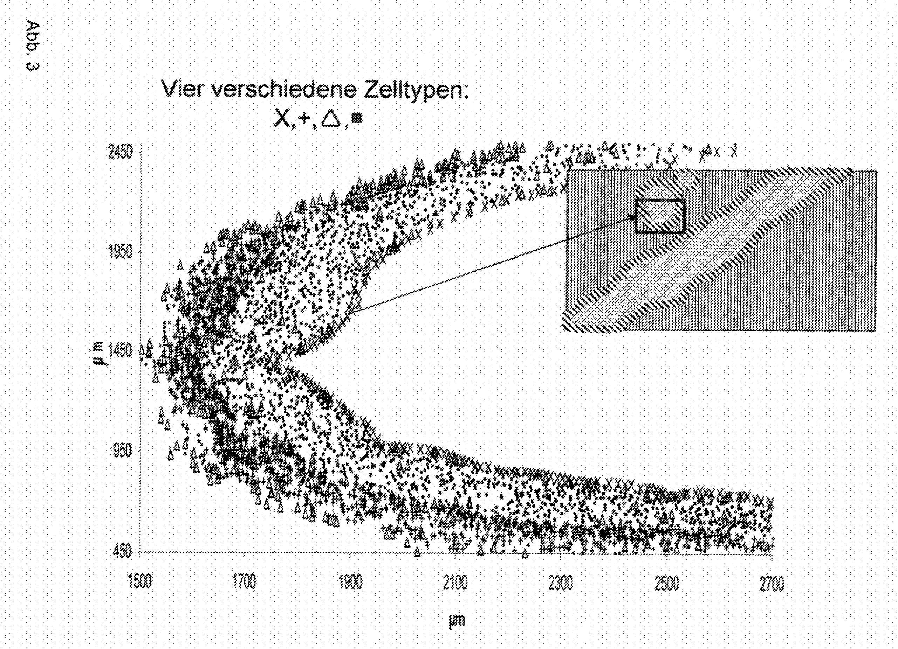

Der Schichtaufbau ist genauer in Abbildung 3 zu sehen. Die einzelnen Flächenbilder 10 werden so geschichtet, dass sich der komplette dreidimensionale Datensatz, hier veranschaulicht in der 3D-Struktur 20, ergibt.

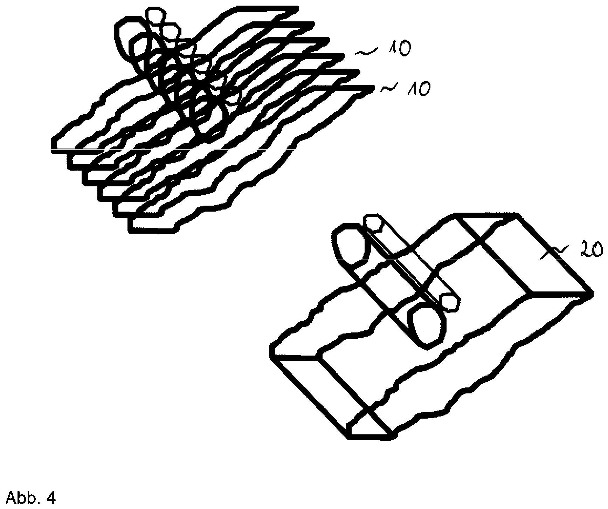

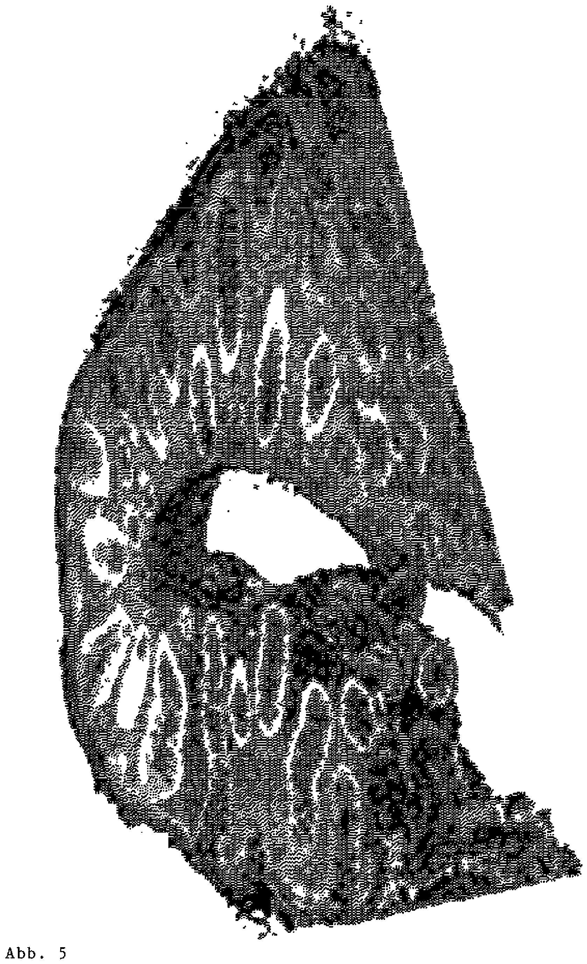

Aus Abbildung 4 ist zu erkennen, wie die Struktur eines histologisch erfassten Organabschnitts im Rechner aussehen kann. Zum Kenntlichmachen der Schnittstruktur wurde eine Organscheibe ausgewählt. In dieser Scheibe sind bereits eine Vielzahl übereinandereschichteter Schnittdaten verwertet. Die erfassten und Rekonstruktionsmaterialien zugeordneten Raumdaten werden für die Steuerung der Prototypingverfahren für die Nachbildung, z.B. ein Funktionsmodell, verwendet, d.h. in den bevorzugten Beispielen für die 2-Photonenpolymerisation von Polymeren für die extrazelluläre Matrix und für den LIFT der verschiedenen Zelltypen. Es können derzeit bis zu drei Zellarten parallel in einem LIFT-Durchlauf positioniert werden.

### BEISPIELE

### 1. Konstruktion eines gefäßdurchzogenen Organabschnitts (nicht erfindungsgemäß):

Zunächst wird die Struktur des in dem nachzubildenden Organteil vorhandenen Gefäßastes festgestellt. Die strukturgeometrischen Daten für den Gefäßast werden für eine 2-Photonenpolymerisation der Stützstruktur des Astes verwendet. Im Beispiel wird diese hohle röhrenartige Struktur, die verzweigt sein kann, aus Polyethylenglycol-Diacrylat oder PEG-Tetraacrylat durch Photopolymerisation in Gegenwart eines Photoinitiators hergestellt. Dieses Verfahren ist als solches bekannt.

Alternativ können die natürlichen Proteine Fibrinogen und Thrombin zu Fibrin polymerisiert werden. Das Verfahren würde dann entsprechend stereolithographisch geführt wie oben grundsätzlich beschrieben.

Die hergestellte Struktur wird gespült.

Anschließend wird die Struktur im Zielbereich einer LIFT-Apparatur so gedreht, dass die Außenseiten des aus dem Polymer erstellten Gefäßastes ortsgenau mit solchen Zellen belegt werden, die aus den strukturgeometrischen Daten für diese Positionen bekannt sind. Diese Prozedur wird solange wiederholt bis der schichtenweise Auftrag von Zellen abgeschlossen und das Organkonstrukt erhalten worden ist. Beispielsweise werden für den Aufbau von Herzbereichen Muskelzellen (Herzmuskelzellen), Bindegewebszellen (Fibroblasten) und Nervenzellen verwendet.

Abschließend werden die Innenflächen des Gefäßastes (die Luminalauskleidung der Gefäße) mit Hilfe eines Beschichtungsverfahrens mit den dort laut Strukturgeometriedaten vorzusehenden Zellen (hier: Endothelzellen) belegt. Hierfür werden diese Zellen in Suspension oder in einem niedrigviskosen Hydrogel in den Gefäßinnenräumen verteilt und die Struktur wird bis zur Anhaftung einer Zellschicht auf der Gefäßinnenseite gedreht oder geschwenkt. Der oben beschriebene Aufbau mit Zellen und ggf. anderen Materialien und die nachfolgende Beschichtung geschieht vorzugsweise immer unter Perfusion, um die frisch aufgebrachten Zellen sofort mit Nährstoffen zu versorgen und Stoffwechselprodukte abzutransportieren. Hierfür wird Blut oder Nährlösung durch die Gefäße des Modells geleitet. Der Vorzug dieses Beispiels, bei dem mit dem Aufbau der Gefäße begonnen wird, liegt demnach auch darin, dass von Beginn an eine Perfusionsmöglichkeit geschaffen wird, die die natürlichen Verhältnisse gut nachmodelliert.

### 2. Aufbau eines Organteils in Schnittebenen entsprechenden Schichten:

Für dieses Beispiel wird das Teilorganmodell in ebenen Schichten aufgebaut, wobei für jede Schicht nacheinander alle dafür notwendigen Rekonstruktionsmaterialien mit ortsselektiven Aufbauverfahren eingebracht werden.

Die Modellierung beginnt an einer mittleren Querschnittsebene des Organs, um eine große Auflagefläche für das sich aufbauende Funktionsmodell zu erhalten. Auf eine Grundplatte aus Glas oder Edelstahl werden mit den ausgewählten Prototyping-Verfahren sowohl Zellen, wie auch Strukturmaterialien, d.h. Polymere bzw. extrazelluläre Matrix, aufgebracht. Dies erfolgt wie bei einem Druck- oder Kopievorgang. Entsprechend den Strukturdaten werden die Zellen jeweils mit dem LIFT-Verfahren "aufgedruckt", d.h. auf der Grundplatte an die für diese Organebene berechnete Position gebracht. Hierfür wird die LIFT-Glasplatte, auf die die zu transferierenden Zellen aufgetragen sind, in definiertem Abstand parallel zur Grundplatte angeordnet. Die Zellbeschichtung befindet sich auf der der Grundplatte zugewandten Seite, der Laser auf der der Grundplatte abgewandten Seite. Nach der Beschichtung mit einer Zellart an den durch die strukturgeometrischen Daten vorgegebenen Positionen wird die Glasplatte für den LIFT-Transport einer anderen Zellart gewechselt.

Für das Matrixmaterial wird das flüssige Vorläufermaterial über die aktuell darzustellende Ebene geschichtet und an den Punkten, an denen Matrixpolymer aufgebracht werden soll, bestrahlt. Danach wird gespült.

Die darauffolgende Schicht des Funktionsmodells wird in gleicher Weise wie oben beschrieben aufgebracht. Schließlich wird das Teilmodell von der Grundplatte abgenommen und gewendet, damit der schichtweise Aufbau in die entgegengesetzte Richtung fortgesetzt werden kann.

Alternativ erfolgt der Aufbau eines Organteils von oben nach unten oder umgekehrt.

Beispielsweise werden für den Aufbau von Lungenbereichen Epithelzellen (Aveolarepithelzellen (Pneumozyten)) und Bindegewebszellen (Chondrozyten und Fibroblasten) verwendet.

## Patentansprüche

1. Verfahren zur Herstellung einer Nachbildung eines menschlichen oder tierischen Organs oder eines Teils davon oder eines sonstigen Segments eines menschlichen oder tierischen Körpers, **gekennzeichnet durch** die Schritte:
a) Bereitstellen vorab erfasster strukturgeometrischer Daten des nachzubildenden Organs, Organteils oder Segments, die einen Datensatz aus einer Angabe zu einem Materialtyp oder einer Materialklasse in Zuordnung zu dessen dreidimensionaler Positionierung im Raum bilden und dadurch Raumkoordinaten für Materialien wie Bindegewebe, Zellen (1, 2), Flüssigkeitsbereiche, Leerräume (3) bereitstellen, wobei die strukturgeometrischen Daten aus angefärbten, vorzugsweise immunhistologisch angefärbten Schnittbildern (10) gewonnen worden sind oder nichtinvasiv am lebenden Körper ermittelt wurden.
b) Auswahl von Rekonstruktionsmaterialien für das nachzubildende Organ oder den nachzubildenden Teil, die wenigstens folgende Gruppen umfassen: wenigstens ein Material zur Rekonstruktion einer extrazellulären Matrix und wenigstens eine Art organspezifischer Zellen (1, 2);
c) Zuordnung der strukturgeometrischen Daten zu den einzelnen gewählten Rekonstruktionsmaterialien auf Basis von Messwerten aus Schnittdarstellungen (10), insbesondere von Farbwerten, Schwärzungsgraden, Messintensitäten oder Kontrastwerten
d) schichtweiser (10) Aufbau des 3D-Modells (20) durch aufeinanderfolgende Anwendung ortsselektiver Aufbauverfahren für jede einzelne Schicht für wenigstens einen Teil der Rekonstruktionsmaterialien, die wenigstens ein Matrixmaterial und wenigstens eine Art organspezifischer Zellen umfassen, anhand eines zugehörigen Datensatzes, wobei ausschließlich ortsselektive Aufbauverfahren angewendet werden oder diese mit weiteren Verfahren wie Beschichtungsverfahren oder Auffüllverfahren kombiniert werden und wobei als ortsselektives Aufbauverfahren für die schichtweise Nachbildung (10) der Zellschichten und -bereiche ein Laser Induced Forward Transfer (LIFT) der Zellen verwendet wird

2. Verfahren nach Anspruch 1, **dadurch gekennzeichnet, dass** aus dem Gesamtdatensatz der strukturgeometrischen Daten einzelne Rekonstruktionsmaterial-spezifische Datensätze für die Konstruktion der Nachbildung gewonnen werden;

3. Verfahren nach Anspruch 1 oder 2, **dadurch gekennzeichnet, dass** zusätzlich manuell festgelegte Raumkoordinaten für die Korrelation verwendet werden.

4. Verfahren nach einem der Ansprüche 1 bis 3, **dadurch gekennzeichnet, dass** als ortsselektives Aufbauverfahren für die schichtweise Nachbildung der extrazellulären Matrix ein Stereolithographieverfahren oder der Laser Induced Forward Transfer (LIFT) angewendet wird.

5. Verfahren nach einem der Ansprüche 1 bis 4, **dadurch gekennzeichnet, dass** der Aufbau in ebenen Schichten (10) oder alternativ in sphärischen Schichten erfolgt.

6. Verfahren nach einem der Ansprüche 1 bis 5, **dadurch gekennzeichnet, dass** der schichtweise Aufbau der Nachbildung ab einer ausgewählten Schnittfläche (10) alternierend oder nacheinander in beide Querrichtungen zur Schnittfläche erfolgt.

7. Verfahren nach einem der Ansprüche 1 bis 6, **dadurch gekennzeichnet, dass** die Lumina (3) zur Stabilisierung mit einem löslichen oder unter milden Bedingungen entfernbaren Polymer gefüllt werden.

8. Verfahren nach einem der Ansprüche 1 bis 7, **dadurch gekennzeichnet, dass** das Material zur Rekonstruktion der extrazellulären Matrix ein durch Energieeinwirkung, vorzugsweise durch Strahlung verfestigbares Polymer ist, welches als solches oder im Gemisch mit ein oder mehreren Biopolymeren oder Biopolymer-mono- oder Oligomeren verwendet wird.

9. Verfahren nach einem der Ansprüche 1 bis 8, **dadurch gekennzeichnet, dass** als Zellen (1; 2) für die organspezifische Nachbildung Zellen der folgenden Gruppe als Rekonstruktionsmaterialien verwendet werden: Typ Epithelzellen, Typ Muskelzellen (1), Typ Bindegewebszellen, Typ Nervenzellen sowie Stammzellen, und dass als weitere Rekonstruktionsmaterialien gegebenenfalls Ionen, Aminosäuren, Peptide, Proteine, Lipide, Kohlenhydrate, Nukleinsäuren, Wachstumsfaktoren, Differenzierungsfaktoren, Nährstoffe, Hormone und/oder Chemotaktika zugefügt werden.

10. Verfahren nach einem der Ansprüche 1 bis 9, **dadurch gekennzeichnet, dass** Zusatzstoffe, wie insbesondere Ionen, Aminosäuren, Peptide, Proteine, Lipide, Kohlenhydrate, Nukleinsäuren, Wachstumsfaktoren, Differenzierungsfaktoren, Nährstoffe, Hormone und/oder Chemotaktika während der Aufbauschritte mit den Zellen (1; 2) eingebracht oder nach den Aufbauschritten oder nach einzelnen Aufbauschritten durch Beschichtungsverfahren und/oder Spülen in die Nachbildung eingebracht werden.

## Claims

1. A method for producing a model of a human or animal organ or a part thereof or of some other segment of human or animal body, comprising the steps of:
a) providing precaptured structural geometric data of the organ, organ part or segment to be replicated which data from a data set composed of information relating to a material type or material class assigned to a three-dimensional positioning in space and, as a result, provide spatial coordinates for materials such as connective tissue, cells (1, 2), fluid regions, empty space (3), wherein the structural geometric data are obtained from stained section images, preferably immunohistologically stained section images (10) or in that the structural geometric data have been determined non-invasively on a living body,
b) selecting reconstitution materials for the organ, organ part or segment to be replicated comprising at least the following groups: at least one material for the reconstruction of an extracellular matrix and at least one type of organ specific cells (1, 2);
c) assigning the structural geometric data to the individual selected reconstitution materials on the basis of measured values from section images (10), particularly, from colour values, degrees of darkness, measured intensities or contrast values;
d) assembling the 3D model (20) layer by layer (10) through successive application of side selective assembly methods for each single layer for at least some of the reconstruction materials, which comprises at least one matrix material and at least one type of organ specific cells, on the basis of an associated data set, wherein only side selective assembly methods are applied or they are combined with further methods such as coating methods or filling methods and wherein the side selective assembly method used for the layer by layer replication (10) of the cellular layers and regions is a laser induced forward transfer (LIFT) of the cells.

2. The method according to claim 1, **characterized in that** individual reconstruction material specific data sets for the construction of the replica obtained from the total data set of the structural geometric data.

3. The method according to claim 1 or 2, **characterized in that** additionally manually determined spatial coordinates are used for the correlation.

4. The method according to any one of claims 1 to 3, **characterized in that** the side selective assembly method applied for the layer by layer replication of the extracellular matrix is a stereolithographic method or laser induced forward transfer (LIFT).

5. The method according to any one of claims 1 to 4, **characterized in that** the assembly is carried out in planar layers (10) or alternatively in spherical layers.

6. The method according to any one of claims 1 to 5, **characterized in that** the layer by layer assembly of the replica is carried out from a selected section surface (10) in both transverse directions with respect to the section surface in an alternate or successive manner.

7. The method according to any one of claims 1 to 6, **characterized in that** the lumina (3) are stabilized by filling them with a soluble polymer or a polymer removable under mild conditions.

8. The method according to any one of claims 1 to 7, **characterized in that** the material for the reconstruction of the extracellular matrix is a polymer solidifiable by energy exposure, preferably by radiation, which is used as such or in a mixture with one or more biopolymers or biopolymer monomers or oligomers.

9. The method according to any one of claims 1 to 9, **characterized in that** as cells (1, 2) for the organ specific replica, cells of the following groups are used as reconstruction materials: epithelial cells, muscle cells (1), connective tissue cells, neurons and stem cells, and **in that** further reconstruction material which may be possibly added are ions, amino acids, peptides, proteins, lipids, carbohydrates, nucleic acids, growth factors, differentiation factors, nutritions, hormones and/or chemotactic agents.

10. The method according to any one of claims 1 to 9, **characterized in that** additives in particular ions, amino acids, peptides, proteins, lipids, carbohydrates, nucleic acids, growth factors, differentiation factors, nutritions, hormones and/or chemotactic factors are introduced into the replica by means of coating methods and/or rinsing after the assembly step or after individual assembly steps or introduced with the cells (1, 2) during the assembly steps.

## Revendications

1. Procédé destiné à la fabrication d'une reproduction d'un organe humain ou animal ou d'une partie d'un organe humain ou animal ou d'un autre segment d'un corps humain ou animal, **caractérisé par** les phases suivantes :
a) la mise à disposition de données géométriques structurales, préalablement déterminées, relatives à l'organe, à la partie d'organe ou au segment à reproduire, lesquelles données forment un lot de données constitué à partir d'une indication relative à un type de matière ou à une catégorie de matière en association avec leur positionnement tridimensionnel dans l'espace, et permettent par conséquent la mise à disposition de coordonnées dans l'espace pour des matières telles que le tissu conjonctif, des cellules (1, 2), des zones de fluide, des espaces vides (3) ;
dans lequel les données géométriques structurales ont été obtenues à partir de vues en coupe (10) colorées, de préférence colorées de manière immunohistochimique ou qui ont été déterminées de manière non invasive sur le corps vivant ;
b) la sélection de matières de reconstruction pour l'organe à reproduire ou pour la partie à reproduire, lesquelles comprennent tout au moins les groupes suivants : tout au moins une matière destinée à la reconstruction d'une matrice extracellulaire et tout au moins une forme de cellules (1, 2) spécifiques à l'organe ;
c) l'association des données géométriques structurales avec les diverses matières de reconstruction sélectionnées sur la base de valeurs de mesure issues de représentations en coupe (10), en particulier de valeurs de couleur, de niveaux de densité, d'intensités de mesure ou de niveaux de contraste ;
d) la réalisation par couches (10) du modèle en 3D (20) par l'application successive de procédés de réalisation à sélectivité de lieu pour chaque couche individuelle pour tout au moins une partie des matières de reconstruction, lesquelles comprennent tout au moins une matière de matrice et tout au moins une forme de cellules spécifiques à l'organe, à l'aide d'un lot de données qui lui est associé ;
dans lequel seuls des procédés de réalisation à sélectivité de lieu sont utilisés ou ceux-ci sont combinés à d'autres procédés, tels que des procédés de revêtement ou des procédés de remplissage ; et
dans lequel un procédé de transfert des cellules induit par laser, dénommé procédé LIFT (Laser Induced Forward Transfer), est utilisé en tant que procédé de réalisation à sélectivité de lieu pour la reproduction (10) par couches des couches de cellules et des zones de cellules.

2. Procédé selon la revendication 1, **caractérisé en ce que** des lots de données individuels, lesquels sont spécifiques à la matière de reconstruction, sont obtenus à partir de l'ensemble des lots de données des données géométriques structurales pour la réalisation de la reproduction.

3. Procédé selon la revendication 1 ou 2, **caractérisé en ce que** des coordonnées dans l'espace qui ont été définies en outre manuellement sont utilisées pour la corrélation.

4. Procédé selon l'une des revendications 1 à 3, **caractérisé en ce qu'**un procédé stéréolithographique ou le procédé de transfert des cellules induit par laser (LIFT) est utilisé comme procédé de réalisation à sélectivité de lieu pour la reproduction par couches de la matrice extracellulaire.

5. Procédé selon l'une des revendications 1 à 4, **caractérisé en ce que** la réalisation est effectuée en couches (10) planes ou, éventuellement, en couches sphériques.

6. Procédé selon l'une des revendications 1 à 5, **caractérisé en ce que** la réalisation par couches de la reproduction est réalisée à partir d'une surface de coupe (10) sélectionnée, en alternance ou de manière consécutive dans les deux directions transversales par rapport à la surface de coupe.

7. Procédé selon l'une des revendications 1 à 6, **caractérisé en ce que** les tubulures (3) destinées à la stabilisation sont remplies d'un polymère soluble ou d'un polymère qui peut être supprimé dans des conditions douces.

8. Procédé selon l'une des revendications 1 à 7, **caractérisé en ce que** la matière destinée à la reconstruction de la matrice extracellulaire est un polymère, lequel peut être fixé sous l'effet de l'énergie, de préférence par rayonnement, et lequel est utilisé en tant que tel ou sous une forme mélangée avec un ou plusieurs biopolymères ou sous une forme mélangée avec un ou plusieurs biopolymères et monomères ou sous une forme mélangée avec un ou plusieurs biopolymères, monomères et oligomères.

9. Procédé selon l'une des revendications 1 à 8, **caractérisé en ce que** les cellules (1, 2) utilisées pour la reproduction spécifique aux organes sont des cellules du groupe suivant qui servent de matières de reconstruction, à savoir du type des cellules épithéliales, du type des cellules musculaires (1), du type des cellules du tissu conjonctif, du type des cellules nerveuses, ainsi que du type des cellules souches ; et
**caractérisé en ce que**, le cas échéant, des ions, des acides aminés, des peptides, des protéines, des lipides, des hydrates de carbone, des acides nucléiques, des facteurs de croissance, des facteurs de différenciation, des nutriments, des hormones et/ou des facteurs chimiotactiques sont ajoutés en tant que matières de reconstruction supplémentaires.

10. Procédé selon l'une des revendications 1 à 9, **caractérisé en ce que** des additifs tels que, en particulier, des ions, des acides aminés, des peptides, des protéines, des lipides, des hydrates de carbone, des acides nucléiques, des facteurs de croissance, des facteurs de différenciation, des nutriments, des hormones et/ou des facteurs chimiotactiques sont introduits avec les cellules (1, 2) pendant les phases de la réalisation, ou sont introduits dans la reproduction après les phases de la réalisation, ou après des phases individuelles de la réalisation, selon un procédé de revêtement et/ou par rinçage.
